# EUROPÄISCHE PATENTANMELDUNG

(11) **EP 4 803 020 A1**
(43) Veröffentlichungstag der Anmeldung: **09.09.2026**
(21) Anmeldenummer: 25162395.5
(22) Anmeldetag: 07.03.2025
(51) Int. Cl.: A61B 18/12, A61B 18/04, H01F 19/04

(54) **MEDIZINISCHES INSTRUMENT MIT BALANCIERTEN ELEKTRODENSTRÖMEN**

(71) Anmelder: Erbe Elektromedizin GmbH, 72072 Tübingen (DE)
(72) Erfinder: DORNHOF, Konstantin, 72160 Horb am Neckar (DE)
(74) Vertreter: Rüger Abel IP Legal Solutions GmbH

(57) **Zusammenfassung**

Zur Bestromung verschiedener Elektroden (18, 19) eines Instruments (10) aus einer zweipoligen Spannungsquelle, z.B. eines Generators 11 dient eine Strombalanciereinrichtung (29), die den von der Stromquelle gelieferten Strom in zwei Teilströme in vorgegebenem Verhältnis aufspaltet. Vorzugsweise ist die Strombalanciereinrichtung (29) eine Wechselstrom-Stromspiegelschaltung mit zwei auf einem gemeinsamen Magnetkreis (37) angeordneten Spulen (L1) und (L2), die gegensinnig von Strom durchflossen werden.

## Beschreibung

Die Erfindung betrifft ein medizinisches Instrument mit balancierten, d.h. aneinander angeglichenen Elektrodenströmen zur elektrochirurgischen Behandlung eines menschlichen oder tierischen Patienten.

Es existieren unterschiedliche Instrumente mit mehreren Elektroden, von denen jeweils ein Strom zur Behandlung biologischen Gewebes ausgeht.

Zum Beispiel offenbaren die EP 3 141 204 B1 und die EP 3 141 203 B1 jeweils ein Instrument zur großflächigen Mukosaablation mit einem Kopf, der zwei Auslassöffnungen mit jeweils einer darin angeordneten Elektrode aufweist. Die Elektroden ionisieren den aus den Öffnungen austretenden Argonstrahl, sodass aus dem Instrument mehrere Plasmastrahlen austreten. Zur Stromversorgung der beiden Elektroden dient ein Generator, der eine hochfrequente Hochspannung erzeugt. Diese wird über eine Schalteranordnung an die beiden Elektroden geleitet. Die Schalteranordnung verbindet die Elektroden in schneller Folge abwechselnd mit dem Generatorausgang, sodass an beiden Elektroden jeweils eine rechteckmodulierte HF-Spannung mit 50% Einschaltdauer ansteht. Jedoch sind die Stromstärken der beiden Elektrodenströme voneinander unabhängig.

Zur Erzeugung mehrerer Plasmastrahlen sieht die US 2005/0015086 A1 ein Instrument mit mehreren Elektroden und Plasmaauslassöffnungen auf. Die Elektroden werden über eine Schaltereinheit von einem Generator gespeist. Die Schaltereinheit dient dazu, aus den vorhandenen Elektroden jeweils eine Teilmenge auszuwählen und mit dem Generator zu verbinden, sodass aus einigen Öffnungen des Instruments Gasstrahlen und aus anderen Öffnungen Plasmastrahlen austreten.

Aus der EP 2 992 849 B1 ist darüber hinaus ein Koagulations-Dissektions-Instrument bekannt, das von einer zangenartigen Grundkonfiguration ausgeht. Die beiden Branchen des zangenartigen Instruments tragen Versiegelungselektroden. Zusätzlich ist eine der beiden Branchen mit einer Schneidelektrode ausgerüstet. Zum Betrieb des Instruments dient ein Generator, der einen zweipoligen Ausgang aufweist. Während einer der Ausgangspole mit einer der Koagulationselektroden verbunden ist, die dann eine Neutralelektrode darstellt, ist der andere Ausgangspol des Generators mit der anderen Versiegelungselektrode der anderen Branche verbunden. Zur Bestromung der Schneidelektrode dient ein Transformator, der in dem Instrument angeordnet sein kann und dazu dient, die von den beiden Polen des Generators abgegebene Spannung auf einen höheren Wert zu transformieren.

In allen genannten Fällen kommt es darauf an, den vom Generator gelieferten Strom auf gewünschte Weise auf die Elektroden des Instruments aufzuteilen. Bei den genannten Plasma-Instrumenten ist zu wünschen, beide Plasma- oder mehrere Plasmastrahlen mit gleichem Strom zu versorgen. Bei dem genannten Koagulations- und Dissektionsinstrument ist zu wünschen den Koagulationsstrom und den Schneidstrom auf zweckentsprechende Weise aufeinander abzustimmen.

Ausgehend davon ist es Aufgabe der Erfindung, ein Instrument anzugeben, das wenigstens zwei mit Strom zu versorgende Elektroden aufweist und bei dem eine gewünschte Aufteilung des von dem Generator gelieferten Stroms auf die beiden Elektroden sichergestellt ist.

Dieser Aufgabe wird mit dem Instrument nach Anspruch 1 gelöst:
Das erfindungsgemäße Instrument weist wenigstens zwei Elektroden auf, die über eine Strombalanciereinrichtung mit nur einem ersten Pol eines zweipoligen Generators verbindbar oder verbunden sind, dessen anderer, zweiter Pol mit einer Neutralelektrode verbunden oder verbindbar ist. Die Strombalanciereinrichtung ist dazu eingerichtet, ein bestimmtes Verhältnis der beiden zu den Elektroden fließenden Ströme einzustellen. Beispielsweise kann der von dem Generator gelieferte Strom gleichmäßig auf zwei (oder mehrere) Elektroden aufgeteilt werden, von denen dann im Wesentlichen gleich starke Plasmastrahlen ausgehen. Auf diese Weise kann mit Instrumenten, die mehrere Plasmastrahlen erzeugen, ein gleichmäßiges Koagulationsergebnis auf biologischem Gewebe erzielt werden.

Die Strombalanciereinrichtung weist vorzugsweise einen mit dem ersten Pol des Generators verbindbaren oder verbundenen Eingang und wenigstens zwei Ausgänge auf, die mit den Elektroden verbunden sind. Es ist prinzipiell möglich, die Strombalanciereinrichtung mit aktiven elektrischen Bauelementen aufzubauen, wie z.B. entsprechend der bekannten Stromspiegelschaltung für Gleichströme. Jedoch ist die Strombalanciereinrichtung vorzugsweise passiv, das heißt ohne Verwendung von aktiven Bauelementen (zum Beispiel Halbleitern) aufgebaut. Dies insbesondere, wenn der Generator ein Hochfrequenzgenerator (HF-Generator) ist, der eine hochfrequente Wechselspannung (HF-Spannung) erzeugt und einen hochfrequenten Strom (HF-Strom) liefert. Die Frequenz des Stroms und der Spannung liegt vorzugsweise zwischen 100 kHz und 1MHz, vorzugsweise bei etwa 350 kHz. Die Strombalanciereinrichtung kann auch als "HF-Stromspiegelschaltung" oder kurz "HF-Stromspiegel" angesehen und bezeichnet werden.

Die passive Strombalanciereinrichtung kann zur Spiegelung der HF-Ströme zwei Spulen aufweisen, die magnetisch miteinander koppelnd angeordnet und dabei gegensinnig gepolt sind. Die erste Spule liegt in der Zuleitung zu der ersten Elektrode und wird von einem ersten HF-Strom durchflossen, während die zweite Spule in der Zuleitung zu der zweiten Elektrode liegt und von einem zweiten HF-Strom durchflossen wird. Die gegensinnige Polung der Spulen bedeutet, dass die Spulen bei betriebsgemäßem Stromdurchfluss in dem gemeinsamen Magnetkreis einander entgegengesetzt gerichtete magnetische Flüsse erzeugen. Durch die gegensinnige Polung der Spulen kommt es zur Stromsymmetrierung auch bei unterschiedlichen elektrischen Lasten an den beiden, oder mehreren, Elektroden. Dies kann beispielsweise bei Schräghaltung des Instruments durch unterschiedliche Längen der aus den beiden Öffnungen austretenden Plasmastrahlen der Fall sein. Besonders vorteilhaft ist es dabei, wenn die den Elektroden vorgeschalteten Spulen einen Koppelfaktor nahe 1, jedenfalls aber größer als 0,9, vorzugsweise größer als 0,95 aufweisen.

Bei der Stromaufteilung für Plasmainstrumente mit zwei Elektroden wird häufig eine gleichmäßige Aufteilung zu je 50% des Stroms auf jede Elektrode gewünscht. In diesem Fall ist es vorteilhaft, wenn die Spulen miteinander übereinstimmende Windungszahlen aufweisen. Sie können auf einen gemeinsamen Kern, insbesondere einen Ferrit-Ringkern oder andere Kerngeometrien aus hochfrequenztauglichem weichmagnetischem Material gewickelt sein. Sind drei oder mehrere Elektroden vorgesehen, ist die Strombalanciereinrichtung dazu eingerichtet, jeder Elektrode einen Anteil des vom Generator gelieferten Stroms zuzuteilen, der Eins geteilt durch die Anzahl der bestromten Elektroden ist.

Es ist auch möglich, für die zwei Spulen verschiedene Windungszahlen vorzusehen, um Ströme in einem anderen Verhältnis als Eins zu Eins auszubalancieren.

Weitere Einzelheiten von Ausführungsformen der Erfindung sind Gegenstand von Ansprüchen, der Beschreibung oder der zugehörigen Zeichnung.

In der Zeichnung sind Ausführungsbeispiele der Erfindung dargestellt. Es zeigen:
Figur 1 Ein erfindungsgemäßes Instrument im Einsatz bei einer Mukosaablation und angeschlossen an einen speisenden Generator,
Figur 2 Das distale Ende des Instruments nach Figur 1, im Längsschnitt,
Figur 3 Eine abgewandelte Ausführungsform des Instruments nach Figur 1 im Längsschnitt seines distalen Endes,
Figur 4 Das Instrument mit Strombalanciereinrichtung, angeschlossen an einen Generator, in schematischer Schaltungsdarstellung,
Figur 5 Die Strombalanciereinrichtung aus Figur 4, in anschaulicher Darstellung,
Figur 6 Ein Koagulations- und Dissektions-instrument angeschlossen an einen speisenden Generator,
Figur 7 Das distale Ende des Instruments nach Figur 6,
Figur 8 Eine Strombalanciereinrichtung zur Speisung der Elektroden des Instruments nach Figur 6 und 7 mit ungleichen Strömen,
Figur 9 Eine Strombalanciereinrichtung zur Speisung von vier Elektroden,
Figur 10 Die Strombalanciereinrichtung für zwei Elektroden in Prinzipdarstellung.

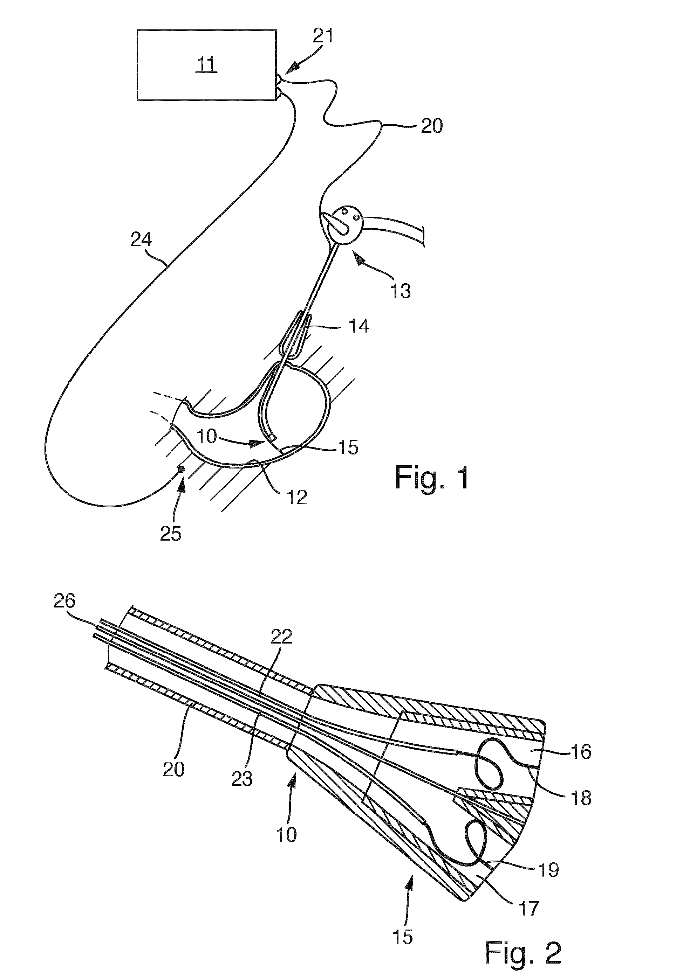

In Figur 1 sind ein als Argon-Plasma-Ablationssonde ausgebildetes Instrument 10 und ein speisender Generator 11 beim endoskopischen Einsatz bei der Ablation der Magenschleimhaut 12 eines nicht weiter veranschaulichten Patienten schematisch dargestellt. Das Instrument 10 wird mittels des Endoskops 13 z.B. durch die Speiseröhre 14 des Patienten in den Magen eingeführt und mittels des Endoskops dort entlang der Magenschleimhaut 12 geführt. An dem in Figur 2 gesondert dargestellten distalen Ende des Instruments 10 ist ein Kopf 15 vorgesehen, an dem zwei Gasauslassöffnungen 16, 17 mit darin angeordneten Elektroden 18, 19 vorgesehen sind. Alternativ können auch mehrere Elektroden vorgesehen sein, die in entsprechenden Gasaustrittsöffnungen angeordnet sind. Weiter ist es möglich, zwei oder mehrere Elektroden in gemeinsamen z.B. schlitzförmigen Gasauslassöffnungen anzuordnen.

Unabhängig von ihrer Anzahl und Form sind die Gasauslassöffnungen 16, 17, über einen Schlauch 20 mit Gas, beispielsweise Argon, gespeist. Dazu erstreckt sich der Schlauch 20 von dem Kopf 15 ausgehend bis zu dem proximalen Ende 21 (Figur 1), wo er über ein geeignetes Anschlussmittel an eine Gasquelle angeschlossen ist. Die Gasquelle kann in dem zur Bestromung der Elektroden 18, 19 vorgesehenen Generator 11 angeordnet sein. Alternativ kann die Gasquelle außerhalb des Generators 11 gesondert bereitgestellt sein, an die der Schlauch 20 angeschlossen ist.

Der Generator 11 dient dazu, die beiden Elektroden 18, 19 mit einer Hochspannung zu versorgen, sodass von beiden Elektroden 18, 19 hochfrequente Ströme ausgehen können. Diese ionisieren den aus den Gasauslassöffnungen 16, 17 austretenden Gasstrom, sodass der Kopf 15 nebeneinander zwei Plasmastrahlen aussendet. Die Elektroden 18, 19 sind dazu über entsprechende elektrische, gegeneinander isolierte Leitungen 22, 23 mit dem Generator 11 verbunden. Dieser enthält (mindestens) einen Oszillator zur Erzeugung einer hochfrequenten Hochspannung und zur Abgabe von hochfrequenten Behandlungsströmen. Die Leitungen 22, 23 erstrecken sich von den Elektroden 18, 19 bis zu dem proximalen Ende 21 des Schlauchs 20, um dort z.B. über das Anschlussmittel mit dem Generator 11 verbunden zu sein. Zwischen den Leitungen 22, 23 und dem Oszillator ist eine Strombalanciereinrichtung 29 angeordnet, die dazu eingerichtet ist, den von dem Oszillator abgegebenen Strom gleichmäßig auf die beiden Elektroden 16, 17 aufzuteilen.

Der Generator 11 ist über eine weitere elektrische Leitung 24 und eine Neutralelektrode 25 mit dem Gewebe des Patienten niederohmig verbunden, um die von den Elektroden 18, 19 ausgehenden Behandlungsströme aufnehmen und zum Generator zurückführen zu können. Die Neutralelektrode kann eine großflächige Klebelektrode sein, die an einer Hautpartie des Patienten anzubringen ist. Sie ist dazu eingerichtet, einen großflächigen elektrischen Kontakt zu dem Patienten zu schaffen. Die Neutralelektrode kann aber auch an dem Instrument selbst angeordnet sein.

An dem Kopf 15 können Sensormittel vorgesehen sein, beispielsweise in Gestalt einer Lichtleitfaser 26, die zur Abstandskontrolle, Spektralanalyse des von den Plasmastrahlen erzeugten Lichts oder dergleichen, genutzt werden kann. Weitere Sensoren sind möglich.

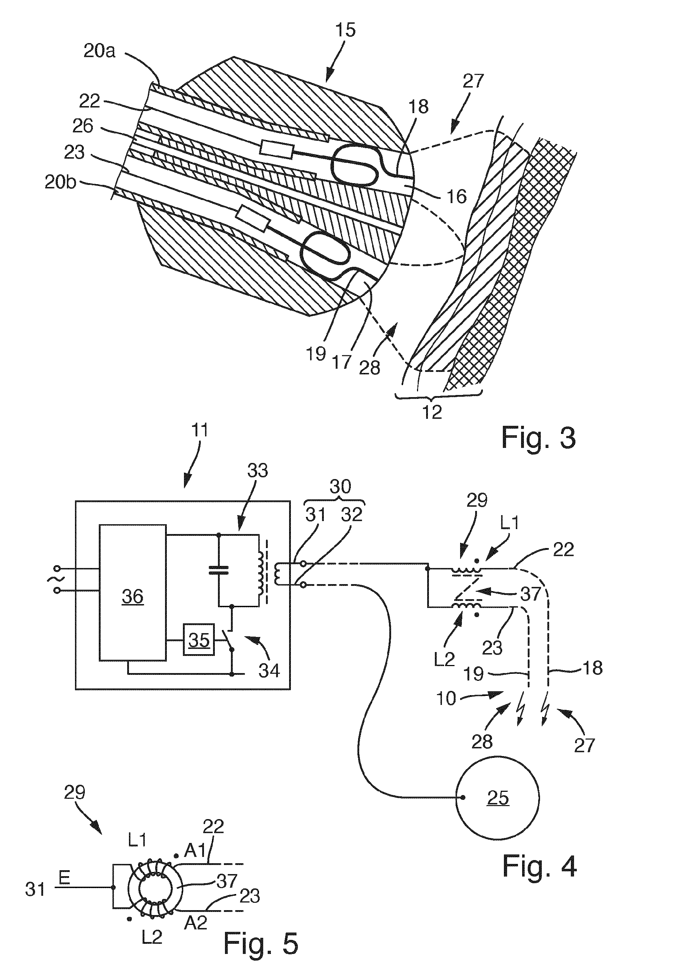

Figur 3 veranschaulicht eine abgewandelte Ausführungsform des als Ablationssonde ausgebildeten Instruments 10 anhand seines Kopfes 15. Für den Kopf 15 nach Figur 3 gilt die vorige Beschreibung entsprechend. Zusätzlich gilt, dass anstelle eines einzigen Schlauchs 20 zur Gaszuführung zwei parallel geführte Schläuche 20a und 20b vorgesehen sind, die den beiden Gasauslassöffnungen 16, 17 individuell zugeordnet sind.

Aus den beiden Gasauslassöffnungen 16, 17 treten Plasmastrahlen 27, 28 aus, die die Magenschleimhaut 12 treffen und dabei möglichst im Wesentlichen gleich wirksam sein sollen. Dazu sind das Instrument 10 und der Generator 11 so beschaffen, dass die von den Elektroden 18, 19 in das Argongas übergehenden HF-Ströme im Wesentlichen gleich groß sind. Dazu ist den Elektroden 18, 19 des als Ablationssonde ausgebildeten Instruments 10 die schon genannte Strombalanciereinrichtung 29 vorgeschaltet, über die die Elektroden 18, 19 mit dem Generator 11 verbunden sind. Die Strombalanciereinrichtung 29 ist eine HF-Stromspiegelschaltung, die dazu eingerichtet ist, die in den Leitungen 22, 23 fließenden HF-Ströme aneinander anzugleichen. Damit wird der von dem Generator 11 an ihren Eingang E gelieferte HF-Strom gleichmäßig auf die beiden Ausgänge A1, A2 aufgeteilt, an die die Elektroden 18, 19 angeschlossen sind. Die Strombalanciereinrichtung 29 kann z.B. in einem Stecker angeordnet sein, der an dem proximalen Ende 21 des Schlauchs 20 angeordnet ist und der zum elektrischen Anschluss des Instruments 10 an den Generator 11 dien.

Der Generator 11 weist zum Anschluss des Instruments 10 typischerweise einen zweipoligen Ausgang 30 auf, zu dem ein erster Pol 31 zur Stromversorgung der Elektroden 18, 19 und ein zweiter Pol 32 zum Anschluss der Neutralelektrode 25 gehören. Zwischen den Polen 31, 32 steht eine hochfrequente Ausgangsspannung an, die aus einem Schwingkreis 33 des Generators 11 ausgekoppelt oder auf andere Weise bereitgestellt sein kann. Zur Anregung des Schwingkreises 33 dienen ein oder mehrere elektronische Schalter 34, die von einer Steuereinrichtung 35 im Takt der zur erzeugenden Hochfrequenzschwingung geöffnet und geschlossen werden können. Zur Energieversorgung dient eine Stromversorgungseinheit 36, die typischerweise netzgespeist ist.

Der aus dem ersten Pol 31 fließende Strom und der in den zweiten Pol 32 fließende Strom sind gleich groß. Der aus dem Pol 31 fließende Strom wird durch die Strombalanciereinrichtung 29 aufgespalten. Die Strombalanciereinrichtung 29 ist dabei dazu eingerichtet, die Ströme in den beiden Leitungen 22 und 23 in einem festen Verhältnis zueinander zu halten, insbesondere in einem Verhältnis von 1:1.

Der aus dem Pol 31 zu der Strombalanciereinrichtung 29 fließende Strom ist ein hochfrequenter Wechselstrom, dessen Frequenz der Schwingfrequenz des Parallelschwingkreises 33 entspricht. Ebenso sind die Ströme in den Leitungen 22 und 23 hochfrequente Wechselströme, deren Stromstärken jeweils halb so groß sind, wie die Stromstärke des aus dem Pol 31 fließenden Stroms.

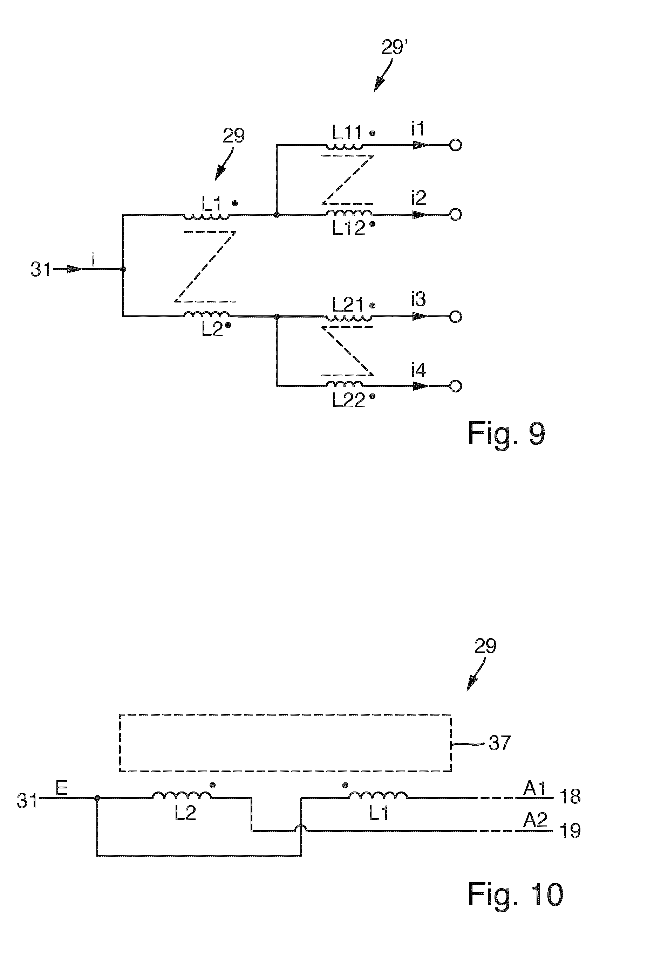

Um eine Angleichung der Ströme in den Leitungen 22, 23 und somit der aus den Elektroden 18, 19 durch die Plasmastrahlen 27, 28 fließenden Ströme zu erreichen, weist die Strombalanciereinrichtung 29 eine erste Spule L1 auf, die den Pol 31 mit der Leitung 22 verbindet. Außerdem weist die Strombalanciereinrichtung 29 eine zweite Spule L2 auf, die den Pol 31 mit der Leitung 23 verbindet. Beide Spulen L1, L2 sind auf einem gemeinsamen Magnetkreis 37 angeordnet, wobei die Spulen L1, L2 gegensinnig gepolt sind. Durch die gegensinnige Polung der Spulen L1 und L2 heben sich die von den jeweils durchfließenden Strömen erzeugten Magnetfelder in dem Magnetkreis 37 gegenseitig auf. Zur Veranschaulichung des Begriffs der "gegensinnigen Polung" der Spulen L1 und L2 wird auf Figur 10 verwiesen. Figur 10 zeigt dazu eine schematisierte Magnetkreisdarstellung mit gleicher Topologie wie die Stromspiegelschaltung 29 nach Figur 4 oder 5. Die Spulen L1 und L2 sind in dem Sinne gegensinnig angeordnet, dass die Spulen L1 und L2 in dem gemeinsamen Magnetkreis einander entgegen gerichtete magnetische Flüsse erzeugen, wenn sie von gleichphasigen elektrischen Strömen durchflossen sind. Au Sicht des Magnetkreises 37 sind die von dem Beiden Spulen erzeugten magnetischen Flüsse um 180° phasenversetzt.

Die von den Spulen induzierten magnetischen Flüsse heben einander auf, wenn die beiden Ströme gleich groß (sowie gleichphasig) sind. Die Spulen L1 und L2 haben dazu gleiche Windungszahlen. Sie sind über den Magnetkreis 37 induktiv miteinander verbunden, wobei der Koppelfaktor der beiden Spulen L1, L2 miteinander vorzugsweise größer als 0,9, weiter vorzugsweise größer als 0,95 ist und im Idealfall nahe 1,0 liegt. In Figur 4 sind die Wicklungsanfänge der Spulen L1 und L2 durch einen Punkt gekennzeichnet. In diesem Fall liegen die Wicklungsanfänge jeweils auf Seiten der Elektroden 18, 19. In der praktischen Ausführung mit Ringkern und gleichsinnig gewickelten Spulen L1, L2 sind die Wicklungsanfänge der Spulen einander entgegengesetzt, das heißt der Wicklungsanfang der Spule L1 ist mit der Leitung 22 verbunden, während der Wicklungsanfang der Spule L2 mit dem Pol 31 verbunden ist.

Die Strombalanciereinrichtung 29 kann Teil des Generators 11 sein, sodass zum Anschluss der Leitungen 22, 23 jeweils eigene Buchsen vorgesehen oder Kontakte vorgesehen sind. Alternativ kann die Strombalanciereinrichtung 29 Teil des Instruments, das heißt der Ablationssonde 10 und dazu beispielsweise in deren Stecker 21 untergebracht sein. Weiter ist es möglich, die Strombalanciereinrichtung 29 als gesonderte Einheit, beispielsweise als Zwischenstecker, auszubilden, dessen Eingang an den Pol 31 und dessen beide Ausgänge an die Leitungen 22, 23 anzuschließen sind.

Die insoweit beschriebene aus dem Generator 11 und dem als Ablationssonde ausgebildeten Instrument 10 bestehende Einheit arbeitet wie folgt:

Es wird davon ausgegangen, dass der Behandler die Ablationssonde, wie in Figur 1 veranschaulicht, in den Patienten eingeführt und in der Nähe der Magenschleimhaut 12 positioniert hat. Soll nun die Ablation begonnen werden, wird zunächst die Gasquelle aktiviert, sodass in dem Schlauch 20 oder in den Schläuchen 20a, 20b ein Gasstrom zustande kommt und aus den Gasauslassöffnungen 16, 17 Gas austritt. Es wird nun der Generator 11 aktiviert, sodass zwischen den beiden Polen 31, 32 eine hochfrequente Ausgangsspannung mit mehreren 100 bis über 1000 Volt ansteht. Bevor sich an den Elektroden 18, 19 elektrische Entladungen bilden, bleibt die Gasstrecke hochohmig, sodass an den Spulen L1, L2 keine Spannung abfällt und auch kein Strom durch sie hindurchfließt.

Zündet nun eine der Elektroden 18, 19, beispielsweise die erste Elektrode 18, entsteht ein aus der Gasauslassöffnung 16 austretender Plasmastrom 27, wobei die Spannung an der Elektrode 18 auf geringe Werte, typischerweise unter 100 Volt, zusammenbricht. Die Differenz zwischen dieser Spannung und der Spannung an dem ersten Pol 31 fällt nun an der Spule L1 ab. Diese induziert in der Spule L2 eine ebenso große Spannung, die sich wegen der gegensinnigen Polung der Spulen L1 und L2 nun zu der Ausgangsspannung des Generators 11 addiert, sodass an der Elektrode 19 eine erhöhte (Zünd-)spannung anliegt und sich somit praktisch sofort eine Plasmaentladung auch an der Elektrode 19 bildet. Es brennen nun die beiden Plasmastrahlen 27, 28, die durch Unregelmäßigkeiten der Magenschleimhaut 12 ebenso wie durch Schrägstellung des Kopfes 15 unterschiedliche Längen haben können. Durch die magnetische Kopplung der beiden Spulen L1 und L2 gleichen sich die in den Leitungen 22, 23 fließenden Ströme an. Jeder durch die Spule L1 fließende Strom induziert in der Spule L2 einen gleich großen Strom. Ebenso induziert jeder in der Spule L2 fließende Strom in der Spule L1 einen gleich großen Strom.

Durch die Angleichung der Stromstärken der den beiden Elektroden 18 und 19 zufließenden Ströme aneinander wird erreicht, dass die beiden Plasmastrahlen 27, 28 an der Schleimhaut 12 gleich starke Koagulationseffekte hervorrufen. So kann der Behandler ohne auf die Einhaltung ganz gleicher Abstände zwischen den Gasaustrittsöffnungen 16, 17 und der Magenschleimhaut 12 achten zu müssen, gleichmäßige Koagulationsergebnisse erzielen.

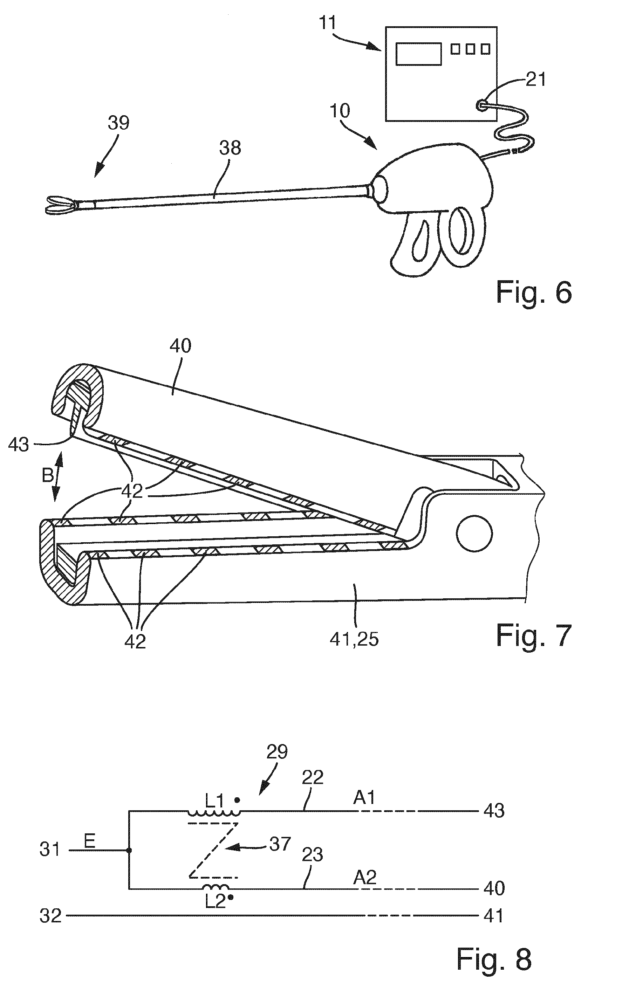

Die vorliegende Erfindung eignet sich nicht nur zur Stromangleichung von Elektroden gleicher Funktion, wie es bei dem als Ablationssonde ausgebildeten Instrument 10 der Fall ist, sondern auch zur Stromangleichung bei Instrumenten mit Elektroden, die unterschiedliche Funktionen aufweisen. Dies wird nachstehend anhand der Figuren 6 bis 8 erläutert:

Das in Figur 6 veranschaulichte Instrument 10 ist ein laparoskopisches Zangeninstrument, an dessen Schaft 38 ein Koagulations- und Dissektionswerkzeug 39 gehalten ist. Dieses weist zwei aufeinander zu- und voneinander weg schwenkbar beweglich gelagerte Branchen 40, 41 mit Elektroden auf, die zur Gewebekoagulation dienen. Dabei können die Branchen 40, 41 an den einander zugewandten Koagulationsflächen isolierende Stellen 42 aufweisen, um elektrische Kurzschlüsse zwischen den Elektroden beim Schließen der Branchen zu vermeiden.

Wie Figur 8 zeigt, kann die Branche 41 beziehungsweise die an ihr angeordnete oder von ihr gebildete Elektrode als Neutralelektrode angesehen werden, die mit dem zweiten Pol 32 des Generators 11 verbunden ist.

In der anderen Branche 40 kann eine Schneidelektrode 43 unbeweglich angeordnet sein, die dazu dient, zwischen den Branchen 40, 41 gefasstes und durch Bestromung koaguliertes Gewebe zu durchtrennen. Dabei kann es erforderlich sein, dass der zu der Koagulationselektrode der Branche 40 geleitete Strom in einem bestimmten Verhältnis zu dem zu der Schneidelektrode 43 geleiteten Strom steht. In diesem Fall kann in dem Gehäuse des Instruments 10 oder auch an anderer Stelle (z.B. im Instrumentenstecker) die Strombalanciereinrichtung 29 gemäß Figur 8 untergebracht sein.

Die Strombalanciereinrichtung 29 enthält, wie schon zuvor beschrieben, zwei über den Magnetkreis 37 miteinander verbundene Spulen L1 und L2. Der Koagulationselektrode der Branche 40 wird der Strom über die Spule L2 zugeführt. Der Schneidelektrode 43 wird der Strom über die Spule L1 zugeführt. In diesem Fall können die Induktivitäten der Spulen L1, L2 und somit deren Windungszahlen unterschiedlich festgelegt sein, beispielsweise um den Schneidstrom an der Schneidelektrode 43 in einem bestimmten Verhältnis zu dem Koagulationsstrom an der Branche 40 festzulegen.

Wiederum ist der Koppelfaktor vorzugsweise größer 0,90, besser 0,95 und bestenfalls nahe 1,0. Außerdem kann vorgesehen sein, dass eine der beiden Spulen L1, L2 (oder auch beide) mit einem willkürlich betätigbaren Schalter überbrückbar sind, um die Induktivität der Spulen L1 und L2 unwirksam zu machen. Unabhängig davon kann vorgesehen sein einen Schalter zur Unterbrechung des Stroms in einer der beiden Spulen L1 oder L2 vorzusehen um gezielt die Schneidelektrode 43 und/oder die an der Branche 40 vorgesehene Koagulationselektrode deaktivieren zu können.

Figur 9 veranschaulicht die Konfiguration einer Strombalanciereinrichtung 29', die zur Aufspaltung eines Stroms i in vier gleich große Teilströme i1 bis i4 dient. Dabei haben die Spulen L1, L2 der ersten Strombalanciereinrichtung 29 übereinstimmende Werte. Ebenso stimmen die Induktivitäten der nachfolgenden Spulen L11 und L12 miteinander überein. Ebenso die Spulen L21, L22. Bei Wahl unterschiedlicher Induktivitäten können die Verhältnisse der Ströme i1 bis i4 zueinander auch von 1:1 abweichend eingestellt werden. Sollte eine ungerade Anzahl von Elektroden zu speisen sein, kann die erste Strombalanciereinrichtung 29 asymmetrisch ausgebildet sein und die Ströme im Verhältnis 1:2 aufteilen. Der stärkere der aufgeteilten Ströme kann dann durch eine weitere Strombalanciereinrichtung 29 im Verhältnis 1:1 aufgeteilt werden. Es ergibt sich so insgesamt eine Stromverteilung der drei Ströme von 1/3 : 1/3 : 1/3.

Zur Bestromung verschiedener Elektroden 18, 19 eines Instruments 10 aus einer zweipoligen Spannungsquelle, z.B. eines Generators 11 dient eine Strombalanciereinrichtung 29, die den von der Stromquelle gelieferten Strom in zwei Teilströme aufspaltet. Vorzugsweise ist die Strombalanciereinrichtung 29 eine Wechselstrom-Stromspiegelschaltung mit zwei auf einem gemeinsamen Magnetkreis 37 angeordneten Spulen L1 und L2, die gegensinnig von Strom durchflossen werden.

### Bezugszeichen:

- 10: Instrument (z.B. Argon-Plasma-Ablationssonde oder Koagulations-Dissektions-Zange)
- 11: Generator
- 12: Magenschleimhaut
- 13: Endoskop
- 14: Speiseröhre
- 15: Kopf
- 16, 17: Gasauslassöffnungen
- 18, 19: Elektroden
- 20: Schlauch
- 20a, 20b: Schläuche
- 21: Anschlussmittel
- 22, 23: elektrische Leitungen zu den Elektroden 18, 19
- 24: elektrische Leitung zu der Neutralelektrode 25
- 25: Neutralelektrode
- 26: Lichtleitfaser
- 27, 28: Plasmastrahlen
- 29, 29': Strombalanciereinrichtung
- E: Eingang der Strombalanciereinrichtung 29
- A1, A2: Ausgänge der Strombalanciereinrichtung 29
- 30: zweipoliger Ausgang
- 31: erster Pol
- 32: zweiter Pol
- 33: Schwingkreis
- 34: elektronischer Schalter
- 35: Steuereinrichtung
- 36: Stromversorgungseinheit
- L1: erste Spule
- L2: zweite Spule
- 37: Magnetkreis
- 38: Schaft
- 39: Koagulations- und Dissektionswerkzeug
- 40, 41: Branche
- 42: isolierende Stelle
- 43: Schneidelektrode

## Patentansprüche

1. Instrument (10) zur medizinischen Behandlung von biologischem Gewebe (12),
mit wenigstens einer ersten und einer zweiten Elektrode (18, 19), die über eine Strombalanciereinrichtung (29) mit nur einem ersten Pol (31) eines Generators (11) mit zweipoligem Ausgang (30) verbindbar oder verbunden sind, dessen anderer, zweiter Pol (32) mit einer Neutralelektrode (25) verbunden oder verbindbar ist.

2. Instrument nach Anspruch 1, **dadurch gekennzeichnet, dass** die Strombalanciereinrichtung (29) einen mit dem ersten Pol (31) verbindbaren oder verbundenen Eingang (E) und wenigstens zwei, jeweils mit einer der wenigstens zwei Elektroden (18, 19) verbundene Ausgänge (A1, A2) aufweist.

3. Instrument nach einem der vorstehenden Ansprüche, **dadurch gekennzeichnet, dass** die Strombalanciereinrichtung (29) eine erste Spule (L1) und eine zweite Spule (L2) aufweist, die magnetisch miteinander koppelnd angeordnet und gegensinnig gepolt sind, indem die erste Spule (L1) und die zweite Spule (L2) in einem gemeinsamen Magnetkreis (37) gegensinnige Felder erzeugend angeordnet sind.

4. Instrument nach einem der vorstehenden Ansprüche, **dadurch gekennzeichnet, dass** die Spulen (L1, L2) bifilar gewickelt sind.

5. Instrument nach einem der Ansprüche 3 oder 4, **dadurch gekennzeichnet, dass** die Spulen (L1, L2) einen Koppelfaktor (K) aufweisen, der größer als 0,9, vorzugsweise größer als 0,95 ist.

6. Instrument nach einem der Ansprüche 3 bis 5, **dadurch gekennzeichnet, dass** die Spulen (L1, L2) miteinander übereinstimmende Windungszahlen aufweisen.

7. Instrument nach einem der Ansprüche 3 bis 6, **dadurch gekennzeichnet, dass** die Elektroden (18, 19) gleich ausgebildet sind.

8. Instrument nach einem der Ansprüche 1 bis 5 oder 7, **dadurch gekennzeichnet, dass** die Spulen (L1, L2) voneinander verschiedene Windungszahlen aufweisen.

9. Instrument nach einem der vorstehenden Ansprüche 1 bis 6 oder 8, **dadurch gekennzeichnet, dass** die Elektroden (18, 19) unterschiedlich ausgebildet sind.

10. Instrument nach einem der vorstehenden Ansprüche, **dadurch gekennzeichnet, dass** das Instrument (10) eine oder mehrere Gasaustrittsöffnungen (16, 17) aufweist, die an einem distalen Ende des Instruments (10) angeordnet sind.

11. Instrument nach einem der vorstehenden Ansprüche, **dadurch gekennzeichnet, dass** die Gasaustrittsöffnung (16) oder die Gasaustrittsöffnungen (16, 17) über eine Gasführung (20, 20a, 20b) des Instruments (10) mit Gas zur Erzeugung eines Plasmastroms (27, 28) versorgbar sind.

12. Instrument nach Anspruch 11, **dadurch gekennzeichnet, dass** die Gasführung (20, 20a, 20b) sich von dem distalen Ende des Instruments (10) bis zu seinem proximalen Ende (21) erstreckend ausgebildet ist.

13. Instrument nach Anspruch 11 oder 12, **dadurch gekennzeichnet, dass** das Instrument (10) an seinem proximalen Ende (21) Anschlussmittel zum Anschluss der Gasführung (20, 20a, 20b) an eine Gasversorgungsquelle aufweist.

14. Instrument nach einem der vorstehenden Ansprüche, **dadurch gekennzeichnet, dass** das Instrument (10) an seinem proximalen Ende (21) Anschlussmittel zum Anschluss des Eingangs der Strombalanciereinrichtung (29) an den Generator (11) aufweist.

15. Instrument nach einem der Ansprüche 10 bis 14, **dadurch gekennzeichnet, dass** die Elektroden (18, 19) in der einen oder in den mehreren Austrittsöffnungen (16, 17) angeordnet sind.
